# EUROPEAN PATENT APPLICATION

(11) **EP 4 190 799 A1**
(43) Date of publication of application: **07.06.2023**
(21) Application number: 21851571.6
(22) Date of filing: 30.07.2021
(51) Int. Cl.: C07K 7/08, A61K 39/215, A61P 31/14, A61P 37/04, C07K 14/165

(54) **AMINO ACID SEQUENCE DERIVED FROM SARS-COV-2 AND USE THEREOF**

(30) Priority: 31.07.2020 JP 2020131022
(71) Applicant: Toagosei Co., Ltd., Minato-ku Tokyo 105-8419 (JP); Kyoto University, Kyoto-shi, Kyoto 606-8501 (JP)
(72) Inventor: YOSHIDA, Tetsuhiko, Tsukuba-shi, Ibaraki 300-2611 (JP); BAILEYKOBAYASHI, Nahoko, Tsukuba-shi, Ibaraki 300-2611 (JP); YOSHIDA, Yoshinori, Kyoto-shi, Kyoto 606-8501 (JP)
(74) Representative: Bals & Vogel Patentanwälte PartGmbB
(86) International application number: PCT/JP2021/028366
(87) International publication number: WO 2022/025264

(57) **Abstract**

The herein disclosed art provides amino acid sequence information that can be used to produce antibodies to protein derived from the novel coronavirus (SARS-CoV-2). Synthetic peptide having the herein disclosed amino acid sequence information is recognized as an antigen in at least one species of mammal; contains any of the following amino acid sequences: (1) MKFLVFLGIITTVAA (SEQ ID NO: 1), (2) MFVFLVLLPLVSSQC (SEQ ID NO: 2), and (3) MKIILFLALITLATC (SEQ ID NO: 3); and has a total number of amino acid residues of not more than 20.

## Description

### Technical Field

The present invention relates to the amino acid sequence information of a SARS-CoV-2-derived protein, that can contribute to the treatment and prevention of the spread of infection by the novel coronavirus (SARS-CoV-2, severe acute respiratory syndrome coronavirus 2). The present invention also relates to the use of this amino acid sequence information.

This application claims priority based on Japanese Patent Application No. 2020-131022 filed on July 31, 2020, the entirety of which is incorporated herein by reference.

### Background Art

SARS-CoV-2 is a virus that infects humans, causing COVID-19 (coronavirus disease 2019). It is a pathogenic virus that rapidly causes symptoms such as severe pneumonia and that can even kill the infected individual. Since the discovery of infection by this virus in the period from December 2019 to early 2020, this infection has spread throughout the world and has become an infectious viral disease that exercises serious effects on the global economy and human lives, just like the previously known SARS and MERS.

The genomic sequence of SARS-CoV-2 is published in Non Patent Literature 1 and can be viewed in a database published by the National Center for Biotechnology Information (NCBI). According to this database, at least 10 genes are predicted to be present on the genome of SARS-CoV-2, and research is being rapidly advanced by researchers in various fields such as virology, genetics, biochemistry, and pharmacology.

### Citation List

### Non Patent Literature

Non Patent Literature 1: Wu, F., et al., Nature, Vol. 579, No. 7798 (2020), pp. 265-269

### Summary of Invention

### Technical Problem

However, the current situation is that the development of vaccines and/or antiviral agents effective against SARS-CoV-2 has not been achieved. As a consequence, the treatment of infected individuals is limited to treatment of the symptoms, and the development of vaccines and antiviral agents against SARS-CoV-2 is an urgent matter. In addition, even if the spread of SARS-CoV-2 infection were to be constrained, the potential exists that additional spread of the infection may be caused by mutation, as with the influenza virus, and thus a variety of vaccines and antiviral agents is desired.

The present invention has therefore been pursued in view of the current situation as described above, and the main object of the present invention is to provide amino acid sequence information for producing antibodies against SARS-CoV-2-derived protein. Specifically, the main object is to provide a synthetic peptide having this amino acid sequence information and to provide a composition comprising this synthetic peptide. An additional and related object is to provide a method for producing anti-SARS-CoV-2 antibodies using said synthetic peptide or composition.

### Solution to Problem

Using SignalP-5.0, a signal peptide prediction software available on the web, the present inventors analyzed the amino acid sequences encoded by the gene sequences present in the genomic sequence of SARS-CoV-2, and as a result found three proteins predicted to have signal peptides. Since signal peptides are regions necessary for viral self-replication, the present inventors hypothesized that there would be a strong possibility that the signal peptide sequences of SARS-CoV-2, which engages in self-replication (that is, causes the spread of the infection), would be conserved. It was therefore thought that, by administering antigen having the amino acid sequence of such a predicted signal peptide to an organism such as a mammal, an anti-SARS-CoV-2-derived signal peptide antibody could be produced for which the epitope is a site where SARS-CoV-2 is unlikely to mutate, and intensive investigations were carried out in this regard. As a result, high-titer antibodies were successfully obtained against the following three amino acid sequences:
(1) MKFL VFLGIITTV AA (SEQ ID NO: 1),
(2) MFVFLVLLPLVSSQC (SEQ ID NO: 2), and
(3) MKIILFLALITLATC (SEQ ID NO: 3),
and the present invention was thereby achieved.

That is, the herein disclosed synthetic peptide is a synthetic peptide that is recognized as an antigen in at least one species of mammal, that contains any of the amino acid sequences in the aforementioned SEQ ID NOs: 1 to 3, and that has a total number of amino acid residues of not more than 20.

In accordance with this constitution, these amino acid sequences are recognized as exogenous foreign material (an antigen) by the mammal, and as a consequence, when such a synthetic peptide is administered to the mammal, antibody is produced for which the synthetic peptide is the antigen.

In addition, the herein disclosed composition comprises a moiety that is recognized as an antigen in at least one species of mammal, wherein the composition is provided with a carrier protein and a synthetic peptide that contains any of the amino acid sequences of SEQ ID NOs: 1 to 3 and that has a total number of not more than 20 amino acid residues.

In accordance with this constitution, the carrier protein, due to its large size and high complexity, can increase the immunogenicity of the synthetic peptide.

In a preferred aspect of the herein disclosed composition, the carrier protein is bonded via a prescribed crosslinking agent to the C-terminal side or N-terminal side of the amino acid sequence given by any of SEQ ID NOs: 1 to 3.

In accordance with this constitution, a state is assumed in which the synthetic peptide resides at a greater distance from the surface of the carrier protein, which can increase the probability of producing an antibody for which the synthetic peptide is the epitope.

A method for producing an anti-SARS-CoV-2 antibody is also provided by the teaching disclosed herein. That is, the herein disclosed method for producing an anti-SARS-CoV-2 antibody uses the amino acid information of any of the SEQ ID NOs: 1 to 3 as an antigen for producing this antibody. Doing this makes it possible to produce an antibody against a predicted signal peptide sequence of a SARS-CoV-2-derived protein.

The amino acid sequence information given in SEQ ID NO: 1 is used in a preferred aspect of the herein disclosed antibody production method. Adopting this makes it possible to produce a particularly high-titer antibody against the predicted signal peptide sequence of a SARS-CoV-derived protein.

### Brief Description of Drawings

[Fig. 1]

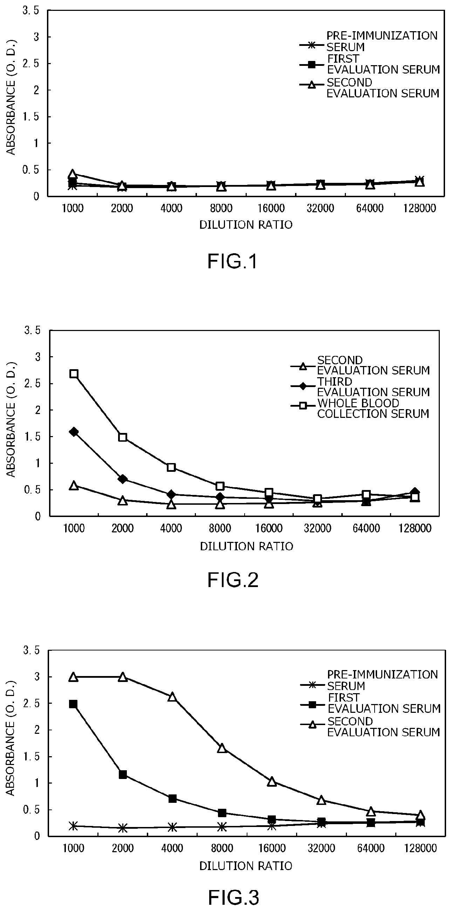

   Fig. 1 is a graph generated by the evaluation of the antibody titer against a synthetic peptide composed of the amino acid sequence of SEQ ID NO: 4, in a pre-immunization serum, first evaluation serum, and second evaluation serum, as obtained by a process of multiple administrations to a first rabbit of a composition provided with keyhole limpet hemocyanin (KLH) and the synthetic peptide composed of the amino acid sequence of SEQ ID NO: 4.
[Fig. 2]
   Fig. 2 is a graph generated by the evaluation of the antibody titer against a synthetic peptide composed of the amino acid sequence of SEQ ID NO: 4, in a second evaluation serum, third evaluation serum, and serum prepared by whole blood collection (whole blood collection serum), as obtained by a process of multiple administrations to the first rabbit of a composition provided with keyhole limpet hemocyanin (KLH) and the synthetic peptide composed of the amino acid sequence of SEQ ID NO: 4.
[Fig. 3]
   Fig. 3 is a graph generated by the evaluation of the antibody titer against a synthetic peptide composed of the amino acid sequence of SEQ ID NO: 4, in a pre-immunization serum, first evaluation serum, and second evaluation serum, as obtained by a process of multiple administrations to a second rabbit of a composition provided with keyhole limpet hemocyanin (KLH) and the synthetic peptide composed of the amino acid sequence of SEQ ID NO: 4.
[Fig. 4]

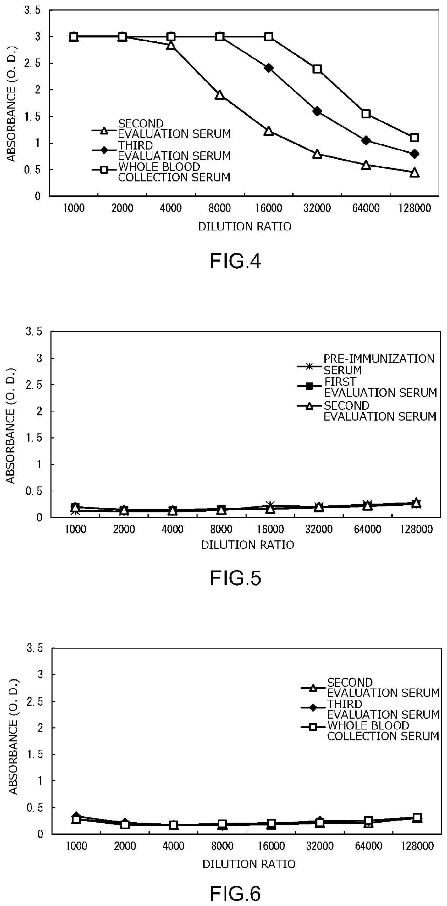

   Fig. 4 is a graph generated by the evaluation of the antibody titer against a synthetic peptide composed of the amino acid sequence of SEQ ID NO: 4, in a second evaluation serum, third evaluation serum, and serum prepared by whole blood collection (whole blood collection serum), as obtained by a process of multiple administrations to the second rabbit of a composition provided with keyhole limpet hemocyanin (KLH) and the synthetic peptide composed of the amino acid sequence of SEQ ID NO: 4.
[Fig. 5]
   Fig. 5 is a graph generated by the evaluation of the antibody titer against a synthetic peptide composed of the amino acid sequence of SEQ ID NO: 2, in a pre-immunization serum, first evaluation serum, and second evaluation serum, as obtained by a process of multiple administrations to a third rabbit of a composition provided with keyhole limpet hemocyanin (KLH) and the synthetic peptide composed of the amino acid sequence of SEQ ID NO: 2.
[Fig. 6]
   Fig. 6 is a graph generated by the evaluation of the antibody titer against a synthetic peptide composed of the amino acid sequence of SEQ ID NO: 2, in a second evaluation serum, third evaluation serum, and serum prepared by whole blood collection (whole blood collection serum), as obtained by a process of multiple administrations to the third rabbit of a composition provided with keyhole limpet hemocyanin (KLH) and the synthetic peptide composed of the amino acid sequence of SEQ ID NO: 2.
[Fig. 7]

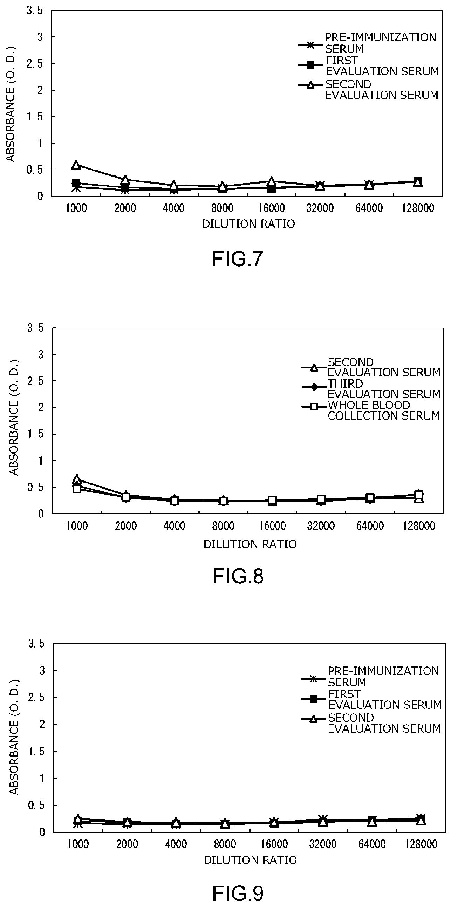

   Fig. 7 is a graph generated by the evaluation of the antibody titer against a synthetic peptide composed of the amino acid sequence of SEQ ID NO: 2, in a pre-immunization serum, first evaluation serum, and second evaluation serum, as obtained by a process of multiple administrations to a fourth rabbit of a composition provided with keyhole limpet hemocyanin (KLH) and the synthetic peptide composed of the amino acid sequence of SEQ ID NO: 2.
[Fig. 8]
   Fig. 8 is a graph generated by the evaluation of the antibody titer against a synthetic peptide composed of the amino acid sequence of SEQ ID NO: 2, in a second evaluation serum, third evaluation serum, and serum prepared by whole blood collection (whole blood collection serum), as obtained by a process of multiple administrations to the fourth rabbit of a composition provided with keyhole limpet hemocyanin (KLH) and the synthetic peptide composed of the amino acid sequence of SEQ ID NO: 2.
[Fig. 9]
   Fig. 9 is a graph generated by the evaluation of the antibody titer against a synthetic peptide composed of the amino acid sequence of SEQ ID NO: 3, in a pre-immunization serum, first evaluation serum, and second evaluation serum, as obtained by a process of multiple administrations to a fifth rabbit of a composition provided with keyhole limpet hemocyanin (KLH) and the synthetic peptide composed of the amino acid sequence of SEQ ID NO: 3.
[Fig. 10]

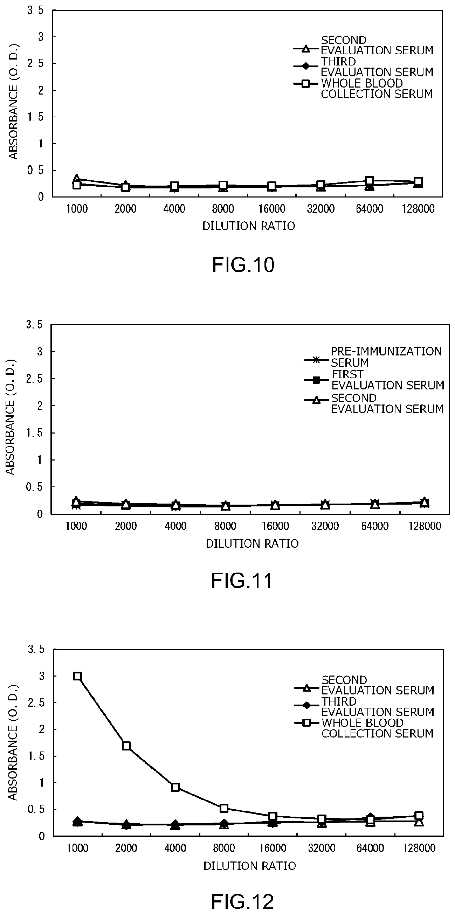

   Fig. 10 is a graph generated by the evaluation of the antibody titer against a synthetic peptide composed of the amino acid sequence of SEQ ID NO: 3, in a second evaluation serum, third evaluation serum, and serum prepared by whole blood collection (whole blood collection serum), as obtained by a process of multiple administrations to the fifth rabbit of a composition provided with keyhole limpet hemocyanin (KLH) and the synthetic peptide composed of the amino acid sequence of SEQ ID NO: 3.
[Fig. 11]
   Fig. 11 is a graph generated by the evaluation of the antibody titer against a synthetic peptide composed of the amino acid sequence of SEQ ID NO: 3, in a pre-immunization serum, first evaluation serum, and second evaluation serum, as obtained by a process of multiple administrations to a sixth rabbit of a composition provided with keyhole limpet hemocyanin (KLH) and the synthetic peptide composed of the amino acid sequence of SEQ ID NO: 3.
[Fig. 12]
   Fig. 12 is a graph generated by the evaluation of the antibody titer against a synthetic peptide composed of the amino acid sequence of SEQ ID NO: 3, in a second evaluation serum, third evaluation serum, and serum prepared by whole blood collection (whole blood collection serum), as obtained by a process of multiple administrations to the sixth rabbit of a composition provided with keyhole limpet hemocyanin (KLH) and the synthetic peptide composed of the amino acid sequence of SEQ ID NO: 3.

### Description of Embodiments

Preferred embodiments of the herein described art are described in the following. Matters required for the execution of the herein disclosed art but not particularly described in this Description (for example, methods for the chemical synthesis of peptides, general matters such as those related to the preparation of compositions) can be understood as design matters for the individual skilled in the art based on the conventional art in the fields of, e.g., cell engineering, physiology, medicine, pharmacology, organic chemistry, biochemistry, genetic engineering, protein engineering, molecular biology, genetics, and so forth. The herein disclosed art can be implemented based on the contents disclosed in this Description and the common general technical knowledge in the pertinent field. In the following text, and depending on the case, the amino acids are represented by the single-letter notation (three-letter notation in the sequence listings) in accordance with the nomenclature for amino acids given in the IUPAC-IUB guidelines.

The contents of all the documents referenced in this Description are incorporated in their entirety in this Description by reference.

In the present Description, a "synthetic peptide" is not a peptide chain that by itself exists independently in nature in a stable manner, but rather refers to a peptide fragment that is produced by artificial chemical synthesis or biosynthesis (i.e., production based on genetic engineering) and is capable of existing stably in a prescribed system (for example, a composition containing an adjuvant).

Herein, "peptide" is a term that denotes an amino acid polymer having a plurality of peptide bonds, and, while not being limited with regard to the number of amino acid residues present in the peptide chain, it typically has a relatively low molecular weight with the total number of amino acid residues being approximately 100 or less (preferably not more than 80, more preferably not more than 70, for example, not more than 60).

Unless specifically indicated otherwise, in the present Description "amino acid residue" is a term that includes the N-terminal amino acid and the C-terminal amino acid of a peptide chain.

"Conjugate" in the present Description is a product (construct) in which the carrier protein and other components (crosslinking agent, Cys residue) are linked by covalent bonding to the synthetic peptide, and is encompassed by the herein disclosed composition.

The amino acid sequences described in this Description always use the N-terminal side for the left side and the C-terminal side for the right side.

The herein disclosed synthetic peptide contains a predicted signal peptide sequence from protein encoded in the SARS-CoV-2 genome published by the NCBI. That is, the synthetic peptide contains any of the following amino acid sequences:
(1) MKFL VFLGIITTV AA (SEQ ID NO: 1),
(2) MFVFLVLLPLVSSQC (SEQ ID NO: 2), and
(3) MKIILFLALITLATC (SEQ ID NO: 3).

The amino acid sequence with SEQ ID NO: 1 corresponds to the predicted signal peptide composed of a total of 15 amino acid residues and deriving from the ORF 8 protein that is a SARS-CoV-2 protein.

The amino acid sequence with SEQ ID NO: 2 corresponds to the predicted signal peptide composed of a total of 15 amino acid residues and deriving from the surface glycoprotein that is a SARS-CoV-2 protein.

The amino acid sequence with SEQ ID NO: 3 corresponds to the predicted signal peptide composed of a total of 15 amino acid residues and deriving from the ORF 7a protein that is a SARS-CoV-2 protein.

These amino acid sequences are sequences that are characteristic of SARS-CoV-2 and as a consequence are readily recognized as an exogenous foreign material (antigen) by organisms, and most prominently mammals, and the production of antibodies against these amino acid sequences can be promoted by the administration of the aforementioned synthetic peptides to organisms and most prominently mammals.

As long as the synthetic peptide contains any of the amino acid sequences of SEQ ID NOs: 1 to 3 and is recognized as an antigen by at least one species of mammal, the synthetic peptide may contain a sequence other than the amino acid sequence of any of SEQ ID NOs: 1 to 3. While not being a particular limitation, for example, the amino acid sequence continuously encoded adjacent to the C-terminal side of the predicted signal peptide of the aforementioned ORF 8 protein may be incorporated on the C-terminal side of the amino acid sequence of SEQ ID NO: 1, and the amino acid sequence continuously encoded adjacent to the C-terminal side of the predicted signal peptide of the aforementioned surface glycoprotein may be incorporated on the C-terminal side of the amino acid sequence of SEQ ID NO: 2. Similarly, the amino acid sequence continuously encoded adjacent to the C-terminal side of the predicted signal peptide of the aforementioned ORF 7a protein may be incorporated on the C-terminal side of the amino acid sequence of SEQ ID NO: 3. Additional examples are sequences provided by the addition of a Cys residue to the C-terminal side or N-terminal side of the amino acid sequence with SEQ ID NO: 1, such as the amino acid sequences with SEQ ID NOs: 4 and 5.

The amino acid sequence with SEQ ID NO: 4 is a sequence provided by the addition of a Cys residue to the C-terminal side of the predicted signal peptide (SEQ ID NO: 1) composed of a total of 15 amino acid residues and derived from the ORF 8 protein that is a SARS-CoV-2 protein.

The amino acid sequence with SEQ ID NO: 5 is a sequence provided by the addition of a Cys residue to the N-terminal side of the predicted signal peptide (SEQ ID NO: 1) composed of a total of 15 amino acid residues and derived from the ORF 8 protein that is a SARS-CoV-2 protein.

The addition of the Cys residue to the C-terminal side or N-terminal side of the amino acid sequence with SEQ ID NO: 1, as in SEQ ID NOs: 4 and 5, makes it possible for the thiol group (SH group) present in the side chain of the Cys residue to react with maleimide that can be present in the crosslinking agent, and thus can facilitate bonding between the synthetic peptide and the crosslinking agent.

The total number of amino acid residues in the herein disclosed synthetic peptide is suitably not more than 20. When greater than this, the potential arises that, upon administration to an organism such as a mammal, antibody will end up being produced for which the epitope is other than the amino acid sequence with any of SEQ ID NOs: 1 to 3. In addition, from the standpoint of limiting the moiety that will form the epitope, the total number of amino acid residues may be not more than 19, or not more than 18, or not more than 17, or not more than 16, and it may be constituted of only the amino acid sequence with any of SEQ ID NOs: 1 to 3.

The amino group at the N-terminal of the herein disclosed synthetic peptide may be N-acetylated. The solubility of the synthetic peptide can be improved by N-acetylation of the N-terminal amino acid residue.

The herein disclosed synthetic peptide can be easily produced in accordance with common chemical synthesis procedures. For example, a heretofore known solid-state synthesis procedure or liquid-phase synthesis procedure may be adopted. A solid-phase synthesis procedure using t-butyloxycarbonyl (Boc) or 9-fluorenylmethoxycarbonyl (Fmoc) as the protecting group for the amino group is favorable.

Alternatively, the synthetic peptide may be biosynthesized based on genetic engineering techniques. That is, a polynucleotide (typically DNA) may be synthesized that has a nucleotide sequence (including the ATG start codon) that encodes the amino acid sequence of the desired synthetic peptide. A recombinant vector may be constructed, in correspondence to the host cell, wherein this recombinant vector has an expressible gene construct comprising the synthesized polynucleotide (DNA) and various regulatory elements (including a promoter, ribosome binding region, terminator, enhancer, and various cis-elements that control expression levels).

Using the usual techniques, this recombinant vector may be introduced into a prescribed host cell (for example, yeast, insect cells, plant cells) and this host cell, or tissue or an individual containing this cell, may be cultured under prescribed conditions. The target peptide can be expressed and produced within the cells as a result. The target antiviral peptide can be obtained by isolating the peptide from the host cells (from the medium when secretion has occurred) and as necessary by carrying out refolding, purification, and so forth.

The methods heretofore used in the relevant fields may be adopted as such for the method for constructing the recombinant vector, the method for introducing the constructed recombinant vector into the host cell, and so forth, and a detailed description of these methods is not provided since these methods as such are not characteristic features of the herein disclosed art.

Alternatively, the target polypeptide can be synthesized in vitro by constructing a template DNA (i.e., a synthetic gene fragment containing a nucleotide sequence that encodes the amino acid sequence of the synthetic peptide) for a cell-free protein synthesis system and using a so-called cell-free protein synthesis system using the various compounds required for peptide synthesis (e.g., ATP, RNA polymerase, amino acids). For information concerning cell-free protein synthesis systems, reference may be made to, for example, the report by Shimizu et al. (Shimizu et al., Nature Biotechnology, 19, 751-755 (2001)) and the report by Madin et al. (Madin et al., Proc. Natl. Acad. Sci. USA, 97(2), 559-564 (2000)). Based on the technology described in these reports, numerous enterprises were already carrying out the contract production of polypeptides at the time this application was filed. Cell-free protein synthesis kits are also commercially available (for example, they are available from CellFree Sciences Co., Ltd. (Japan)).

The single-strand or double-strand polynucleotide containing the nucleotide sequence coding for the herein disclosed synthetic peptide and/or containing the nucleotide sequence complementary to this sequence, can be easily produced (synthesized) by heretofore known methods. Thus, the nucleotide sequence corresponding to the amino acid sequence of the synthetic peptide may be readily determined and provided by selecting the codons that correspond to the individual amino acid residues that constitute the designed amino acid sequence. Once the nucleotide sequence has been determined, the polynucleotide (single strand) corresponding to the desired nucleotide sequence can be easily obtained using, for example, a DNA synthesizer. Using the obtained single-strand DNA as a template, the target double-strand DNA can be obtained using various enzymatic synthesis means (typically PCR). The polynucleotide may take the form of DNA or RNA (e.g., mRNA). The DNA can be provided as the double strand or single strand. When provided as the single strand, it may be the coding strand (sense strand) or may be the noncoding strand (antisense strand) with the sequence complementary to the coding strand.

The thusly obtained polynucleotide can be used as a starting material for the construction of a recombinant gene (expression cassette) for producing the synthetic peptide, as described above, in various host cells or using a cell-free protein synthesis system.

The herein disclosed synthetic peptide is recognized as an antigen in at least one species of mammal and can promote the production of antibodies (e.g., IgM, IgG) that recognize this synthetic peptide. The synthetic peptide may take the form of a salt as long as it can be recognized as an antigen in at least one species of mammal. For example, use can be made of the acid-addition salt of this peptide, as may be obtained by carrying out an addition reaction with a commonly used inorganic acid or organic acid using a common method. Alternatively, other salts (for example, metal salts) may be used as long as recognition as an antigen in at least one species of mammal occurs. The "peptide" referenced in this Description and the instant claims encompasses peptides in this salt form.

The herein disclosed synthetic peptide is also provided as a portion of a composition that has a carrier protein. That is, the herein disclosed composition is a composition comprising a moiety that is recognized as an antigen in at least one species of mammal, wherein the composition comprises a carrier protein and a synthetic peptide having a total number of not more than 20 amino acid residues, wherein the synthetic peptide contains any of the following amino sequences:
(1) MKFL VFLGIITTV AA (SEQ ID NO: 1),
(2) MFVFLVLLPLVSSQC (SEQ ID NO: 2), and
(3) MKIILFLALITLATC (SEQ ID NO: 3).

The species of the carrier protein is not particularly limited, but, for example, any of the following antigenic stimulants can be advantageously used: keyhole limpet hemocyanin (KLH), ovalbumin (OVA), bovine serum albumin (BSA), and so forth.

In a preferred embodiment of the herein disclosed composition, the carrier protein is bonded via a prescribed crosslinking agent to the C-terminal side or N-terminal side of the synthetic peptide.

Crosslinking agents having homobifunctional groups or heterobifunctional groups as generally used for the crosslinking of peptides can be used as the instant crosslinking agent. Preferred reactive functional groups in this crosslinking agent can be exemplified by various amino-containing compounds (for example, primary amines), thio-containing groups, other sulfur-containing groups, carboxyl, and hydroxyl. The reactive functional group can be specifically exemplified by N-hydroxysuccinimide-activated esters (NHS esters), maleimide, azido, iodoacetamide, and so forth. The NHS esters efficiently react with amine at pH values at and above neutrality and can form a very stable amide bond. Maleimide engages in an SH group selective reaction and at neutrality exhibits a better reaction with the SH group than with amine.

Advantageous crosslinking agents having homobifunctional groups can be exemplified by N-hydroxysuccinimide (NHS), disuccinimidyl suberate (DSS), bis(sulfosuccinimidyl) suberate (BS3), dithiobis(succinimidyl propionate) (DSP), dithiobis(sulfosuccinimidyl propionate) (DTSSP), ethylene glycol bis(succinimidyl succinate) (EGS), ethylene glycol bis(sulfosuccinimidyl succinate) (sulfo-EGS), disuccinimidyl tartrate (DST), and disulfosuccinimidyl tartrate (sulfo-DST). In particular, bis(sulfosuccinimidyl) suberate (BS³) can preferably be used.

The following can preferably be used as advantageous crosslinking agents having heterobifunctional groups: polyethylene glycol (PEG) derivatives, e.g., O-[N-(3-maleimidopropionyl)aminoethyl]-O'-[3-(N-succinimidyloxy)-3-oxopropyl] heptacosaethylene glycol, as well as m-maleimidobenzoyl-N-hydroxysuccinimide ester (MBS), succinimidyl 4-[maleimidophenyl]butyrate (SMPB), succinimidyl 4-(maleimidomethyl)cyclohexane-1-carboxylate (SMCC), N-(y-maleimidobutyroxy)succinimide ester (GMBS), m-maleimidopropionic acid N-hydroxysuccinimide ester (MPS), and N-succinimidyl (4-iodoacetyl)aminobenzoate (SIAB). In particular, PEG provided with NHS ester and maleimide for the reactive functional groups can be preferably used. In addition to improving the solubility in water and human body fluids, PEG has the property of exhibiting almost no immunogenicity. Moreover, PEG is resistant to decomposition in the fluids of the human body. Due to this, a conjugate having a PEG-containing crosslinking agent can be advantageously use as an antigen for administration to an organism.

The location where the synthetic peptide and crosslinking agent are bonded is not particularly limited, but the N-terminal side or C-terminal side is preferred. By doing this, the synthetic peptide assumes a state of greater separation from the surface of the carrier protein and the probability of the production of an antibody for which the synthetic peptide is the epitope can thereby be increased.

While not being a particular limitation, when the crosslinking agent is bonded to the N-terminal side of the synthetic peptide, for example, a reaction can be efficiently carried out between the N-terminal amino group of the synthetic peptide and an NHS ester present in the crosslinking agent, as long as an amino group is not present in the synthetic peptide sequence other than the N-terminal amino group (i.e., as long as no Lys residue is present). In addition, because the thiol group of the Cys residue selectively reacts with maleimide present in the crosslinking agent, the location of bonding with the crosslinking agent can then be made the N-terminal or C-terminal when the Cys residue is present at only the N-terminal or C-terminal of the synthetic peptide. A Cys residue may be added to the N-terminal or C-terminal when the Cys residue is not present, as is the case for the amino acid sequence given by SEQ ID NO: 1. A maleimide-containing crosslinking agent can then be bonded to the N-terminal or C-terminal.

The herein disclosed composition or synthetic peptide containing an amino acid sequence given by any of SEQ ID NOs: 1 to 3 can be used to produce anti-SARS-CoV-2 antibody. The use of the composition or synthetic peptide containing the amino acid sequence given by SEQ ID NO: 1 is preferred thereamong. The composition and synthetic peptide containing the amino acid sequence given by SEQ ID NO: 1 exhibit particularly high antigenicities in mammals and can be used to produce antibodies at higher antibody titers.

A vaccine is an example of a representative method for using the herein disclosed synthetic peptide and composition, and the herein disclosed synthetic peptide and composition can be used, inter alia, as a therapeutic drug or for vaccination against SARS-CoV-2. In another representative example, antibody that recognizes the synthetic peptide is produced by administering the synthetic peptide or composition as an antigen to an organism such as a mammal.

Experimental animals such as guinea pigs, rats, mice, rabbits, sheep, and so forth may be used as the mammal to be immunized, while rats, mice, and rabbits are advantageous for obtaining monoclonal antibodies or polyclonal antibodies. The immunization method may use any route of administration, e.g., subcutaneous, intraperitoneal, intravenous, intramuscular, intradermal, and so forth, but subcutaneous, intradermal, intraperitoneal, or intravenous injection is primarily preferred. In addition, various strategies can be used without particular limitation with regard to the immunization interval, immunization amount, and so forth. For example, in a widely used method, immunization is performed about 2 to 10 times at 2-week intervals, and, after the final immunization, a sample is collected from the organism about 1 to 5 times preferably after about 2 to 7 days. With regard to the immunization dose, the amount of peptide per administration is not limited, but, for example, the use of about 50 to 300 µg per rabbit is preferred. In addition, while not constituting a particular limitation, monoclonal antibody or polyclonal antibody to the synthetic peptide can be efficiently obtained at high antibody titers by carrying out a first administration, intraperitoneally into a mouse, of the synthetic peptide/carrier protein conjugate thoroughly mixed with an adjuvant (for example, Freund's complete adjuvant (FCA)) and bringing about cell growth; carrying out additional intraperitoneal administrations on a two-week interval of the conjugate thoroughly mixed with an adjuvant (e.g., FCA or Freund's incomplete adjuvant (FIA)); and collecting the ascites. Purification of the target monoclonal antibody or polyclonal antibody can be performed by known methods, e.g., affinity chromatography, ion-exchange chromatography, gel filtration, salting out with ammonium sulfate, and so forth.

As long as the synthetic peptide is recognized as an antigen by at least one species of mammal, the herein disclosed synthetic peptide and composition may contain any of various pharmaceutically (medicinally) acceptable carriers in correspondence to the form of use. For example, the carriers commonly used in peptide drugs as, e.g., diluents, excipients, and so forth, can be used.

While the carrier can vary as appropriate in correspondence to the application and form of the carrier-containing synthetic peptide and composition, representative examples of the carrier are water, physiological buffers, and various organic solvents. In addition, the carrier can be an aqueous alcohol (e.g., ethanol) solution of suitable concentration, glycerol, or a nondrying oil such as olive oil. Or it may be a liposome. Examples of secondary components that can be incorporated in the composition are various fillers, extenders, binders, moisturizers, surfactants, colorants, fragrances, adjuvants, and so forth.

Representative forms of the carrier-containing synthetic peptide and composition can be exemplified by solutions, suspensions, emulsions, aerosols, foams, granules, powders, tablets, capsules, ointments, and aqueous gels. In order to support use for, e.g., injection, a lyophilizate or granulate may also be made in order to be able to prepare a drug solution by dissolution in, for example, physiological saline or an appropriate buffer (for example, PBS), immediately before use.

The processes as such for preparing the various composition (drug) forms using the synthetic peptide (main component) and various carriers (secondary component) as starting materials may conform to heretofore known methods, and these formulation methods are not themselves characteristic features of the herein disclosed art and are thus not described in detail. A source of detailed information on formulation is, for example, Comprehensive Medicinal Chemistry, Corwin Hansch, Editor, Pergamon Press (1990). The contents of this publication are incorporated in their entirety in this Description by reference.

Several test examples pursuant to the herein disclosed art are described in the following, but this should not be construed as limiting the herein disclosed art to what is indicated in these test examples.

### [Table 1]

**Table 1**

| Sample No. | amino acid sequence | total number of amino acid residues |
|---|---|---|
| 1 | **MKFLVFLGIITTVAAC** (SEQ ID NO: 4) | 16 |
| 2 | **MFVFLVLLPLVSSQC** (SEQ ID NO: 2) | 15 |
| 3 | **MKIILFIALITLATC** (SEQ ID NO: 3) | 15 |

### < Peptide synthesis >

Peptides composed of the amino acid sequences given in Table 1 were each produced using a commercial peptide synthesizer. The specifics are given in the following.

Sample 1 is a synthetic peptide with the amino acid sequence given in SEQ ID NO: 4, and is a synthetic peptide generated by the addition of the Cys residue to the C-terminal side of the amino acid sequence with SEQ ID NO: 1.

Sample 2 is a synthetic peptide with the amino acid sequence given by SEQ ID NO: 2.

Sample 3 is a synthetic peptide with the amino acid sequence given by SEQ ID NO: 3.

The peptides in samples 1 to 3 were all synthesized by carrying out a solid-state synthesis method (Fmoc method) using a commercial peptide synthesizer in accordance with the accompanying manual. Because the mode of use of the peptide synthesizer per se is not a characteristic feature of the herein disclosed art, a detailed description thereof is not provided. The amino group of the N-terminal amino acid in samples 1 and 2 was acetylated.

### < Conjugate construction using the peptides >

A conjugate was constructed by bonding the sample 1 synthetic peptide to keyhole limpet hemocyanin (KLH), i.e., a carrier protein, using as the crosslinking agent a polyethylene glycol (PEG) having maleimide at one terminal and NHS ester at the other terminal. Because the reactions that bond these together are not themselves a characteristic feature of the herein disclosed art, a detailed description thereof is not provided. However, a brief description is as follows: the conjugate was constructed by bonding the crosslinking agent to the surface of the KLH by reaction of the NHS ester in the crosslinking agent with the amino groups present in the KLH, and the particular synthetic peptide and the KLH were then bonded to each other via the PEG by the reaction with bond formation between the maleimide in the crosslinking agent and the thiol group of the Cys residue present on the C-terminal side of the synthetic peptide. Conjugates with the synthetic peptides of samples 2 and 3 were constructed using the same method as for sample 1.

### < Antibody production using the conjugate as antigen >

Antibody against the synthetic peptide of sample 1 was produced by administering the conjugate of sample 1 to two Japanese white rabbits (when distinguishing the individual rabbits in the following, these rabbits are also referred to as the first rabbit and the second rabbit, respectively). Blood was collected as appropriate from these rabbits in order to evaluate the antibody titer as described below. Specifically, on day 1 (the number of days is indicated in the following with reference to this day), a 5 mL of blood sample was collected prior to the administration of the conjugate of sample 1 and pre-immunization sera were prepared. On the same day, a composition provided by mixing 0.15 mg of the conjugate of sample 1 and an equal volume of Freund's complete adjuvant (FCA) was intradermally administered to the rabbits (1st intradermal administration). On day 15, a composition provided by mixing 0.3 mg of the conjugate of sample 1 mixed with an equal volume of FCA was administered intradermally to the rabbits (2nd administration). On day 29, a composition provided by mixing 0.3 mg of the conjugate of sample 1 with an equal volume of FCA was administered intradermally to the rabbits (3rd administration). On day 36, a 5-mL blood sample was collected from the rabbits and first evaluation sera were prepared. On day 43, a composition provided by mixing 0.3 mg of the conjugate of sample 1 with an equal volume of FCA was administered intradermally to the rabbits (4th administration). On day 50, a 5-mL blood sample was collected from the rabbits and second evaluation sera were prepared. On day 57, a composition provided by mixing 0.3 mg of the conjugate of sample 1 with an equal volume of FCA was administered intradermally to the rabbits (5th administration). On day 64, a 5 mL of blood sample was collected from the rabbits and third evaluation sera were prepared. On day 71, a composition provided by mixing 0.3 mg of the conjugate of sample 1 with an equal volume of FCA was administered intradermally to the rabbits (6th administration). On day 78, whole blood collection was performed on the rabbits and whole blood collection sera were prepared. The prepared pre-immunization sera, first through third evaluation sera, and whole blood collection sera were preserved with the addition of 0.09% sodium azide at the time of preparation.

Using the same procedure as for the conjugate of sample 1, pre-immunization sera, first through third evaluation sera, and whole blood collection sera were also prepared by the administration of the conjugate of sample 2 to two rabbits and the administration of the conjugate of sample 3 to two rabbits. In order to distinguish the individual rabbits, the two rabbits receiving the conjugate of sample 2 are respectively labeled the third rabbit and the fourth rabbit, and the two rabbits receiving the conjugate of sample 3 are respectively labeled the fifth rabbit and the sixth rabbit.

### < Evaluation of the serum antibody titers >

The antibody titers of the pre-immunization sera, first through third evaluation sera, and whole blood collection sera were evaluated using the enzyme-linked immunosorbent assay (ELISA) method.

First, the sample 1 synthetic peptide was dissolved at 5 µg/mL in pH 7.2 PBS (phosphate buffered saline); 100 µL of the mixture solution was added to each well of an Immunoplate; and incubation was carried out at room temperature for 2 hours. The liquid in each well was then removed and the wells were subsequently washed three times with PBS containing 0.2% Tween-20 (polyoxyethylene(20) sorbitan monolaurate, FUJIFILM Wako Pure Chemical Corporation) (wash solution). The wash solution was then added to each well and incubation was carried out overnight at 4°C. After incubation, the pre-immunization sera, first through third evaluation sera, and whole blood collection sera for sample 1 were each diluted 1000-fold, 2000-fold, 4000-fold, 8000-fold, 16000-fold, 32000-fold, 64000-fold, and 128000-fold using PBS containing 0.05% Tween-20 (diluent). After the wash solution had been removed from each well, 100 µL of each dilution was added to separate wells and incubation was carried out at 37°C for 30 minutes followed by incubation for an additional 15 minutes at room temperature. The liquid was removed from each well; washing was then performed three times using the aforementioned wash solution; HRP-conjugated goat f(ab)'2 anti-rabbit IgG (MP Biomedicals, LLC-Cappel Products), diluted 5000-fold with the aforementioned diluent, was added to each well in an amount of 100 µL each; and incubation was carried out at 37°C for 30 minutes followed by incubation for an additional 15 minutes at room temperature. The liquid in each well was removed; washing was then performed 3 times with the aforementioned wash solution; a substrate solution was prepared by dissolving 10 mg of o-phenylenediamine (OPD, purchased from SIGMA) in 25 mL of citrate-phosphate buffer and adding 5 µL of hydrogen peroxide; and 100 µL of the substrate solution was added to each well. Color development was carried out at room temperature for 20 minutes; 100 µL of 1 M sulfuric acid was added to each well to stop color development; and the absorbance of each well at 490 nm was measured using an Immno Reader. The results are shown in Figs. 1 to 4. Figs. 1 and 2 show the ELISA results for the sera from the first rabbit, and Figs. 3 and 4 show the ELISA results for the sera from the second rabbit.

The antibody titers in the pre-immunization sera, first through third evaluation sera, and whole blood collection sera were also evaluated for samples 2 and 3 using the same procedures as for the evaluation of the antibody titers in the sera for sample 1. The results are given in Figs. 5 to 12. Figs. 5 and 6 give the ELISA results for the sera obtained from the third rabbit, and Figs. 7 and 8 give the ELISA results for the sera obtained from the fourth rabbit. Figs. 9 and 10 give the ELISA results for the sera obtained from the fifth rabbit, and Figs. 11 and 12 give the ELISA results for the sera obtained from the sixth rabbit.

In this Description, the evaluation is made that an antibody titer is securely expressed (a good antibody titer is present) when the absorbance at 490 nm exceeds 0.5. In addition, when this absorbance exceeds the measurement upper limit for the Immno Reader (when the absorbance exceeds 3.0), this is reported as an absorbance of 3.0.

As shown in Figs. 1 and 2, in the first rabbit the antibody titer against the sample 1 synthetic peptide was increased by the repeated administration of the sample 1 conjugate, and, because the absorbance substantially exceeded 0.5, it could be confirmed that sera were obtained that contained IgG antibody for which a satisfactory antibody titer was secured.

As shown in Figs. 3 and 4, in the second rabbit the antibody titer against the sample 1 synthetic peptide was increased by the repeated administration of the sample 1 conjugate, and, because the absorbance substantially exceeded 0.5, it could be confirmed that sera were obtained that contained IgG antibody for which a satisfactory antibody titer was secured.

As shown in Figs. 5 and 6, even with repetitive administration of the sample 2 conjugate, the third rabbit was unable to produce a serum for which an antibody titer against the sample 2 synthetic peptide was secured. However, as shown in Figs. 7 and 8, in the fourth rabbit sera with an absorbance exceeding 0.5 could be obtained by the repetitive administration of the sample 2 conjugate. As a consequence, it was confirmed that administration of the sample 2 conjugate produced sera that contained IgG antibody for which an antibody titer against the sample 2 synthetic peptide was secured.

As shown in Figs. 9 and 10, even with repetitive administration of the sample 3 conjugate, the fifth rabbit was unable to produce a serum for which an antibody titer against the sample 3 synthetic peptide was secured. However, as shown in Figs. 11 and 12, in the sixth rabbit a result substantially exceeding an absorbance of 0.5 could be obtained for the whole blood collection serum by the repeated administration of the sample 3 conjugate. As a consequence, it was confirmed that administration of the sample 3 conjugate produced serum that contained IgG antibody for which an antibody titer against the sample 3 synthetic peptide was secured.

When these results are compared, the result is that, among the amino acid sequences of samples 1 to 3, antibody was obtained in a particularly high antibody titer by the administration to the rabbit of a conjugate having a synthetic peptide composed of the sample 1 amino acid sequence. It is thus shown that antibody can be produced in a higher antibody titer by using the amino acid sequence with SEQ ID NO: 1.

Specific examples of the herein disclosed art have been described in detail in the preceding, but these are nothing more than examples and do not limit the claims. Various modifications and alterations of the specific examples provided as examples in the preceding are encompassed by the art described in the claims.

### Industrial Applicability

As has been described in the preceding, the administration to a mammal of the herein disclosed synthetic peptide and composition (conjugate) can produce in the mammal a high titer of antibody that recognizes the synthetic peptide. As a consequence, the synthetic peptide and composition provided by the present teaching can be used as a vaccine against SARS-CoV-2. In addition, the produced antibody can be an antiviral agent against SARS-CoV-2 and can also be used, inter alia, for the detection or labelling of SARS-CoV-2.

## Claims

1. A synthetic peptide that is recognized as an antigen in at least one species of mammal, wherein the synthetic peptide contains any of the following amino acid sequences:
(1) MKFL VFLGIITTV AA (SEQ ID NO: 1),
(2) MFVFLVLLPLVSSQC (SEQ ID NO: 2), and
(3) MKIILFLALITLATC (SEQ ID NO: 3),
and has a total number of not more than 20 amino acid residues.

2. A composition comprising a moiety that is recognized as an antigen in at least one species of mammal, wherein the composition comprises a carrier protein and a synthetic peptide having a total number of not more than 20 amino acid residues, wherein the synthetic peptide contains any of the following amino sequences:
(1) MKFL VFLGIITTV AA (SEQ ID NO: 1),
(2) MFVFLVLLPLVSSQC (SEQ ID NO: 2), and
(3) MKIILFLALITLATC (SEQ ID NO: 3).

3. The composition according to claim 2, wherein the carrier protein is bonded via a prescribed crosslinking agent to the C-terminal side or N-terminal side of the amino acid sequence given by any of SEQ ID NOs: 1 to 3.

4. A method for producing an anti-SARS-CoV-2 antibody, wherein the antibody production method uses the amino acid information of any of the following amino acid sequences:
(1) MKFL VFLGIITTV AA (SEQ ID NO: 1),
(2) MFVFLVLLPLVSSQC (SEQ ID NO: 2), and
(3) MKIILFLALITLATC (SEQ ID NO: 3).
as an antigen for producing the antibody.

5. The method for producing the antibody according to claim 4, that uses the amino acid information of SEQ ID NO: 1.
